# EUROPEAN PATENT SPECIFICATION

(11) **EP 4 135 522 B1**
(45) Date of publication and mention of the grant of the patent: **29.01.2025**
(21) Application number: 21721587.0
(22) Date of filing: 14.04.2021
(51) Int. Cl.: A01N 63/00, A01N 59/00, A01P 1/00

(54) **KIT COMPRISING TWO OR THREE SOLID COMPOSITION FOR PRODUCING ANTIBACTERIAL, ANTIVIRAL, ANTIFUNGAL AND DISINFECTANT SOLUTIONS**
KIT BESTEHEND AUS ZWEI ODER DREI FESTEN ZUSAMMENSETZUNGEN ZUR HERSTELLUNG VON ANTIBAKTERIELLEN, ANTIVIRALEN, FUNGIZIDEN UND DESINFIZIERENDEN LÖSUNGEN
KIT INCLUANT DEUX OU TROIS COMPOSITIONS SOLIDES POUR LA PRODUCTION DE SOLUTIONS ANTIBACTÉRIENNES, ANTIVIRALES, ANTIFONGIQUES ET DÉSINFECTANTES

(30) Priority: 14.04.2020 GB 202005432
(43) Date of publication of application: 22.02.2023
(73) Proprietor: Qures Group Ltd, Bishops Waltham SO32 1AX (GB)
(72) Inventor: STEAD, Richard, Winchester Hampshire SO23 9LS (GB)
(74) Representative: Haseltine Lake Kempner LLP
(86) International application number: PCT/GB2021/050900
(87) International publication number: WO 2021/209753

(56) References cited:
- EP-A1- 1 068 871
- WO-A1-2006/138271
- WO-A1-97/42825
- WO-A1-98/42370
- WO-A1-99/08531
- JP-A- H06 245 779
- US-A1- 2009 246 146
- US-A1- 2014 255 382
- DEY SUBARNA ET AL: "Two linkers are better than one: enhancing CO 2 capture and separation with porous covalent triazine-based frameworks from mixed nitrile linkers", JOURNAL OF MATERIALS CHEMISTRY A, vol. 5, no. 7, 1 January 2017 (2017-01-01), GB, pages 3609 - 3620, XP093191430, ISSN: 2050-7488, DOI: 10.1039/C6TA07076K

## Description

### FIELD OF THE INVENTION

The present invention relates to solid compositions which can be added to an aqueous system to produce antibacterial, antiviral, antifungal and disinfectant solutions.

### BACKGROUND

Enzymes and enzymatic systems are well-known as natural antimicrobials. An example of these are the naturally occurring peroxidase systems which have antimicrobial properties. However, peroxidases alone have no antimicrobial effect. The complete antimicrobial peroxidase system requires three components: a peroxidase enzymatic catalysing agent, hydrogen peroxide and an oxidizable substrate such as a negatively charged halogen or pseudohalogen. The peroxidase-catalyzed oxidation of (pseudo)halogens yields reactive agents which oxidize microorganisms, damaging essential structural and functional components and cause inhibition of microbial metabolism and growth.

Different peroxidases preferably oxidize different halogens or (pseudo)halogens, generating distinct antimicrobial species. For example, myeloperoxidase (MPO) of neutrophils employs chloride as a substrate and forms hypochlorous acid as the main product. Lactoperoxidase (LPO) of milk and salivary peroxidase (SPO) of saliva readily oxidize thiocyanate (SCN-) and generate hypothiocyanous acid or its conjugate base hypothiocyanite (OSCN-), the latest being predominant in most physiological fluids. Iodide (I-) can also be oxidized by MPO, LPO, and SPO and it is the most readily oxidizable of all halides in vitro.

Such peroxidase systems (and those derived from plants) are well-known, and are highly desirable in producing antibacterial, antiviral, antifungal and disinfectant solutions. However, due to the chemical instability of the chemical species involved, the uses of such systems are often limited and storage for extended periods of time is often not possible. Such peroxidase-based products often suffer from the following problems:
- A short shelf life
- A short half-life of the solution containing the active species
- Difficult preparation methods
- The requirement for coagulant (typically a metallic salt) in the product to stabilize and/or separate the suspension in water. Such coagulants (such as aluminium-based coagulants) are highly undesirable if using such a product in contact with food
- Manufacturing costs to make such products are often high

The present invention has been devised in light of the above considerations.

WO 2006/138271 discloses antimicrobial solutions which comprise Lactoperoxidase, a peroxide source, a halide or a thiocyanate and optionally an ammonium source.

WO 99/08531 relates to an antimicrobial composition comprising a haloperoxidase, a hydrogen peroxide source, a halide source and an ammonium source. The Examples test the antibacterial activity of haloperoxidases.

WO 97/42825 relates to an enzymatic antimicrobial composition comprising or consisting essentially of a peroxidase obtainable from or produced by the fungus Coprinus, an enhancing agent, and hydrogen peroxide or a source of hydrogen peroxide.

### SUMMARY OF THE INVENTION

The present invention provides a kit comprising:
a first solid composition comprising:
   (a) a peroxidase enzymatic catalysing agent, wherein the peroxidase enzymatic catalysing agent is selected from Lactoperoxidase, Myeloperoxidase, Eosinophil peroxidase, Urea peroxidase and plant-derived peroxidases;
   (b) an oxidizable substrate selected from:
      (i) chlorides such as sodium chloride, iodides such as potassium iodide and sodium iodide; or
      (ii) sodium thiocyanate, potassium thiocyanate, sodium bisulfite, sodium hydrosulfite, sodium metabisulfite, sodium nitrite, potassium nitrite, sodium hypochlorite and combinations thereof;
   (c) optionally at least one inert filler; and
   (d) a buffer system, wherein the buffer system is selected from citric acid: trisodium citrate; calcium lactate:citric acid; sodium L (+)-tartrate dehydrate: citric acid; calcium lactate : DL-malate : malic acid and Sodium L (+)- tartrate : tartaric acid
a second solid composition comprising:
   (e) at least one oxidising agent, wherein the oxidising agent is selected from calcium peroxide, sodium peroxide, sodium percarbonate and the combination of dextrose and glucose oxidase; and
   (f) optionally at least one inert filler.

In another aspect of the present invention there is provided:
a kit comprising:
a first solid composition comprising:
   (a) a peroxidase enzymatic catalysing agent, wherein the peroxidase enzymatic catalysing agent is selected from Lactoperoxidase, Myeloperoxidase, Eosinophil peroxidase, Urea peroxidase and plant-derived peroxidases;
   (b) optionally at least one inert filler,
a second solid composition comprising:
   (c) an oxidizable substrate selected from:
      (i) chlorides such as sodium chloride, iodides such as potassium iodide and sodium iodide; or
      (ii) sodium thiocyanate, potassium thiocyanate, sodium bisulfite, sodium hydrosulfite, sodium metabisulfite, sodium nitrite, potassium nitrite, sodium hypochlorite;
   (d) optionally at least one inert filler;
   (e) a buffer system, wherein the buffer system is selected from citric acid: trisodium citrate; calcium lactate:citric acid; sodium L (+)-tartrate dehydrate: citric acid; calcium lactate : DL-malate : malic acid and Sodium L (+)- tartrate : tartaric acid and
a third solid composition comprising:
   (f) at least one oxidising agent, wherein the oxidising agent is selected from calcium peroxide, sodium peroxide, sodium percarbonate and the combination of dextrose and glucose oxidase; and
   (g) optionally at least one inert filler.

There is also provided an aqueous system comprising the solid composition as described herein and water. The aqueous system can be used as an antibacterial solution, disinfectant, antifungal solution and antiviral solution. The aqueous system can be used in methods of cleaning, washing and disinfecting materials including surfaces.

The aqueous systems can be used in the preparation of solutions intended for food, pharmaceutical and cosmetic products. Additionally, they can be used to spray into the air and on to surfaces to kill pathogens. For example, the aqueous solution can be sprayed in enclosed spaces or defined open areas, such as in hospitals, for examples, wards, waiting areas, clinical settings and inside airplanes or storage areas for perishable goods to kill airborne pathogens and pathogens on the surfaces.

In another aspect of the present invention there is provided a method of sterilising water comprising the steps of:
using the kit described herein, wherein the first and second solid compositions are added to the water to be sterilised.

In a further aspect of the present invention there is provided a method of preparing an antibacterial solution, disinfectant, antifungal solution and antiviral solution comprising the steps of:
using the kit as defined herein, wherein the first, second and optionally third solid compositions are added to water; and
optionally adding further other anti-infectious, antimicrobial, antiviral, antibiotic, antifungal agents, preservatives or disinfection agents.

In a further aspect, there is also provided a method for using the antibacterial solution, disinfectant, antifungal solution or antiviral solution described herein for:
(i) cleaning, washing and disinfecting materials including surfaces, equipment and medical devices;
(ii) the preparation of solutions intended for pharmaceutical and cosmetic products;
(iii) introducing into the air and applying on to surfaces to kill pathogens; and
(iv) creating fine spray or mist prior to inhalation by human and/or animals.

In a further aspect, there is provided a method of using the antibacterial solution, disinfectant antifungal solution or antiviral solution described herein, alone or in combination with other anti-infectious, antimicrobial, antiviral for the removal of pathogens in agriculture in the form of a spray or mist on fields of flowers or crops or used in greenhouses or used in storage facilities for perishable goods.

This invention includes the combination of the aspects and preferred features described except where such a combination is clearly impermissible or expressly avoided.

### DETAILED DESCRIPTION OF THE INVENTION

Aspects and embodiments of the present invention will now be discussed with reference to the accompanying figures.
Figure 1 is a graph showing a 5 Litre scale-up experiment which shows the amount of hypothiocyanate ions present during 120 hour storage.
Figure 2 is a graph showing the amount of hypothiocyanate ions present with the use of calcium peroxide and sodium percarbonate during 24 hour storage.
Figure 3 is a graph showing the amount of hypothiocyanite ions present with the use of calcium peroxide (buffered with citric acid : trisodium citrate buffer solution) compared to calcium peroxide (non-buffered) and sodium percarbonate (non-buffered) during 24 hour storage.
Figure 4 is a graph showing ppm changes during the final 15-minute mixing of the reaction (post calcium peroxide/ sodium percarbonate addition).
Figure 5 is a graph showing the pH changes through the mixing period.

The composition according to the present invention is in solid form. By this, we mean that the components which form the composition are solid, and preferably are dry.

By peroxidase enzymatic catalysing agents we mean free enzymes which are widely available in nature. Their primary function is to catalyse oxidation reactions whilst consuming oxidative agents, such as hydrogen peroxide. An electron donor (reducing agent) is generally required in order for the oxidation reaction to go forward.

Peroxidases which can be used include peroxidases of plant origin, including but not limited to ascorbate peroxidase and thiol-based peroxidase. According to the present invention, peroxidases are derived from animal or human origin including Lactoperoxidase, Thyroid peroxidase, Myeloperoxidase, Eosinophil peroxidase and Urea peroxidase.

Peroxidase in the presence of hydrogen peroxide and in the presence of halides or thiocyanates as electron donors can generate products that possess a wide range of antimicrobial properties. Peroxidases can vary with respect to the particular halides or thiocyanates with which they can react. For example, myeloperoxidase utilizes Cl", Br", I", or SCN" as the electron donor, and oxidizes them to form antimicrobial hypohalides or hypothiocyanites. Lactoperoxidase catalyzes the oxidation of Br", I", or SCN", but not CI", to generate antimicrobial products. Horseradish peroxidase uses only I" as the electron donor to yield I2, HIO, and IO".

Preferably, the peroxidase used is Lactoperoxidase.

The enzyme may be immobilized or non-immobilized. By the term "immobilized" we mean an enzyme attached to an inert, insoluble material (for example, calcium alginate). This can provide increased resistance to changes in conditions such as pH or temperature. By the term "non-immobilized" we mean that the enzyme is free.

The solid composition comprises an oxidizable substrate. This will be selected based on the peroxidase used. The skilled person is aware of peroxidase systems and understands that if Lactoperoxidase is selected then the oxidizable substrate will either be thiocyanate ions or iodide ions (or mixtures thereof). In another example, if Myeloperoxidase is used then either thiocyanate ions, iodide ions or the chloride ions (or mixtures thereof) are required.

The oxidizable substrate is selected from: negatively charged halogens and their derivatives, or pseudohalogens and their derivatives. The term negatively charged halogen refers to chlorides and iodides. Although bromides can be used, it is preferred that they are not. Pseudohalogens are polyatomic analogues of halogens, whose chemistry resembles true halogens and allows them to substitute for halogens in several classes of chemical compounds. Pseudohalogens occur in pseudohalogen molecules. Examples of pseudohalogens include hypothiocyanite, isothiocyanate and thiocyanate. By derivatives we mean salts thereof.

According to the present invention, the oxidizable substrate is selected from:
(i) halogens such as chlorides such as sodium chloride, iodides such as potassium iodide and sodium iodide; or
(ii) pseudohalogen salts selected from sodium thiocyanate, potassium thiocyanate, sodium bisulfite, sodium hydrosulfite, sodium metabisulfite, sodium nitrite, potassium nitrite, sodium hypochlorite.

Preferably, the oxidizable substrate is selected from sodium thiocyanate, potassium thiocyanate and potassium iodide and combinations thereof.

Optionally, the oxidizable substrate may be encapsulated. Optionally, the oxidizing agent may be encapsulated. Typical encapsulating agents may be used including but not limited to alginate, chitosan, carrageenan, gums (such as xanthan gum) and gelatin. Encapsulation techniques are well known and available to the person skilled in the art.

The oxidizing agent is any chemical compound which is capable of producing hydrogen peroxide. For this invention, the oxidizing agent is in solid form. The oxidizing agent is selected from metal peroxides such as calcium peroxide, magnesium peroxide or sodium peroxide. The oxidizing agent may also selected from permanganates and percarbonates, such as sodium percarbonate.

The oxidising agent may also be the combination of dextrose and glucose oxidase. These produce hydrogen peroxide and a side product glucono delta lactone. The acidity from the glucono delta lactone may be advantageous in that it reduces the pH. At lower pH the active agent created is considered to be hypothiocyanous acid and it is also considered that this has stronger antimicrobial effect than the derivative. At the pH optimum of 5.3, the hypothiocyanate ion is in equilibrium with hypothiocyanous acid. The uncharged hypothiocyanous acid is considered to be the greater bactericidal of the two forms. (Thomas EL, Pera KA, Smith KW, Chwang AK (February 1983). "Inhibition of Streptococcus mutans by the lactoperoxidase antimicrobial system". Infect. Immun. 39 (2): 767-78. PMC 348016. PMID 6832819).

Preferably the oxidizing agent is selected from calcium peroxide and sodium percarbonate. The present Applicant has found that both calcium peroxide and sodium percarbonate, when dissolved in water, release 25 to 30% active oxygen (similar to the availability when hydrogen peroxide is used directly). Notably, both calcium peroxide and sodium percarbonate are available as food approved ingredients.

In accordance with the present invention, the solid composition may also include at least one inert filler which is selected from microcrystalline cellulose, calcium carbonate, dextrose monohydrate, magnesium stearate, dicalcium phosphate, lactose powder, multifunctional starch, partially depolymerized cellulose, partially pre-gelatinized starches, highly functional starch, bentonite and combinations thereof.

By inert filler (or excipient) we mean inactive chemical substances which are used to bulk up solid formulations that contain one or more potent active ingredient(s). The Applicant has found that in a compositions which contain oxidizable substrates such as thiocyanate, there has been an increase in moisture content which may result in clumping. This can make it difficult to dissolve the composition in water. To overcome this, an inert filler may be used. Furthermore, the inert filler is used because accurate filling of capsules and sachets at low levels are not always reliable.

Preferably, the inert filler is microcrystalline cellulose (MCC). This type of inert filler is useful because it is both food and pharma grade approved. Additionally, it absorbs the moisture. Other similar types of inert filler can also be used.

Optionally, the inert filler is silica. This type of inert filler is also useful for the purposes of moisture absorption, thus keeping the solid composition components drier and maintaining stability of the compounds.

In another aspect of the invention, no inert filler is present.

In accordance with the present invention, the solid composition further comprises a buffer system. A buffer is a solution that resists dramatic changes in pH. Buffers do so by being composed of certain pairs of solutes, for example a weak acid plus a salt derived from that weak acid or a weak base plus a salt of that weak base. Buffers are well known in the art. By the term "buffer system" this is meant to describe the solid buffer composition which comprises, for example, an acid and a salt such as citric acid and trisodium citrate. When the buffer system is added to water, the buffer system forms a buffer solution.

Types of salts which may be present in the buffer system include, but are not limited to citrates, borates, carbonates and phosphates. Salts of other organic acids may also be used, including but not limited to acetic acid, malic acid, lactic acid and tartaric acid.

Preferably, the buffer system allows the pH of the solid composition prepared as a solution to fall within less than or equal to 6.5 in the initial stages followed by a rise to a pH of greater than or equal to 7.5. The low pH is optimal for the initial production of hypothiocyanite ions whilst the higher pH then stabilises the hypothiocyanite ions produced. The addition/use of a buffer to stabilise and control pH results in a high initial value of hypothiocyanite, and also stabilises the hypothiocyanite in solution for several hours.

The buffer system is selected from the following systems:
citric acid : trisodium citrate
calcium lactate :citric acid
sodium L (+)-tartrate dehydrate : citric acid;
calcium lactate : DL-: malic acid
Sodium L (+)- tartrate : tartaric acid.

In some examples, the buffer system is present in an amount of from 0.5 to 0.9 grams. The amount of buffer system may be varied depending on the specific components selected and to fit the end use of the desired product.

In some examples, the composition further comprises an inert filler which is hygroscopic and therefore reduced the sensitivity of the buffer system to any moisture present. In some examples, the inert filler is silica.

Preferably, the solid composition does not comprise:
(i) a coagulant;
(ii) a thickening agent, preferably wherein the solid composition does not contain a clay such as bentonite; and/or
(iii) a flocculant.

These types of components are known to be used in peroxidase systems in order to support the reaction medium, but which is then isolated and removed at the end of the reaction. Coagulants such as polyaluminium chloride are typically used. However, such metal-containing coagulants are undesirable when it is possible that the solution which contains them could come into contact with food or drink. Also, it avoids metal contamination of water if/when the water enters the sewage system.

Typical coagulants which are avoided in this invention include aluminium or iron salts including but not limited to aluminium chloride, ferric chloride and polyhydroxychloride aluminium.

Typical flocculants which are avoided in this invention include anionic or cationic polymeric flocculants such as polysaccharides or polyacrylamides. The Applicant has found that the use of flocculants (as well as thickeners) can result in a reduction of the active species (such as hypothiocyanite).

Although it is possible to use bentonite as the inert filler, typical thickening agents are avoided in this invention. Preferably, thickening agents such as clays, kaolin, silica or silicates are not present in the present invention. The Applicant has found that thickeners such as bentonite can often lead to a coloured precipitate which is not desirable when added to water, especially if it is to be added to drinking water.

In a preferred aspect, the present invention comprises:
(a) Lactoperoxidase,
(b) sodium thiocyanate and/or potassium thiocyanate and/or potassium iodide;
(c) calcium peroxide;
(d) microcrystalline cellulose; and
(e) a buffer system.

The buffer system is in accordance with the buffers systems described herein. Preferably, the buffer system is a citric acid : trisodium citrate buffer system.

According to the present invention there is provided a kit comprising:
a first solid composition comprising:
   (a) a peroxidase enzymatic catalysing agent, wherein the peroxidase enzymatic catalysing agent is selected from Lactoperoxidase, Myeloperoxidase, Eosinophil peroxidase, Urea peroxidase and plant-derived peroxidases;
   (b) an oxidizable substrate selected from:
      (i) chlorides such as sodium chloride, iodides such as potassium iodide and sodium iodide; or
      (ii) sodium thiocyanate, potassium thiocyanate, sodium bisulfite, sodium hydrosulfite, sodium metabisulfite, sodium nitrite, potassium nitrite, sodium hypochlorite and combinations thereof;
   (c) optionally at least one inert filler; and
   (d) a buffer system, wherein the buffer system is selected from citric acid: trisodium citrate; calcium lactate : citric acid; sodium L (+)-tartrate dehydrate: citric acid; calcium lactate : DL-malate : malic acid and Sodium L (+)- tartrate : tartaric acid
a second solid composition comprising:
   (e) at least one oxidising agent, wherein the oxidising agent is selected from calcium peroxide, sodium peroxide, sodium percarbonate and the combination of dextrose and glucose oxidase; and
   (f) optionally at least one inert filler.

The kit according to the present invention comprises:
a first solid composition comprising:
   (a) a peroxidase enzymatic catalysing agent in an amount of from 5% to 45% by wt% of the first solid composition;
   (b) an oxidizable substrate in an amount of from 5% to 55% by weight selected from:
      (i) negatively charged halogens and their derivatives, or
      (ii) pseudohalogens and their derivatives; and
   (c) optionally at least one inert filler in an amount of from 0% to 90% by wt of the first solid composition , and
a second solid composition comprising:
   (d) at least one oxidising agent in an amount of from 5% to 100% by wt of the second solid composition; and
   (e) optionally at least one inert filler in an amount of from 0% to 95% by wt of the second solid composition.

If the first solid composition comprises a buffer system then it may be present in an amount of up to 40% by wt of the first composition and the amount of inert filler is reduced accordingly to up to 40% by wt of the first composition.

According to the present invention there is provided a kit comprising:
A kit comprising:
a first solid composition comprising:
   (a) a peroxidase enzymatic catalysing agent, wherein the peroxidase enzymatic catalysing agent is selected from Lactoperoxidase, Myeloperoxidase, Eosinophil peroxidase, Urea peroxidase and plant-derived peroxidases;
   (b) optionally at least one inert filler,
a second solid composition comprising:
   (c) an oxidizable substrate selected from:
      (i) chlorides such as sodium chloride, iodides such as potassium iodide and sodium iodide; or
      (ii) sodium thiocyanate, potassium thiocyanate, sodium bisulfite, sodium hydrosulfite, sodium metabisulfite, sodium nitrite, potassium nitrite, sodium hypochlorite;
   (d) optionally at least one inert filler;
   (e) a buffer system, wherein the buffer system is selected from citric acid: trisodium citrate; calcium lactate : citric acid; sodium L (+)-tartrate dehydrate: citric acid; calcium lactate : DL-malate : malic acid and Sodium L (+)- tartrate : tartaric acid and
a third solid composition comprising:
   (f) at least one oxidising agent, wherein the oxidising agent is selected from calcium peroxide, sodium peroxide, sodium percarbonate and the combination of dextrose and glucose oxidase; and
   (g) optionally at least one inert filler.

According to the present invention there is provided a kit comprising:
a first solid composition comprising:
   (a) a peroxidase enzymatic catalysing agent in an amount of from 3% to 100% by weight of the first solid composition;
   (b) optionally at least one inert filler in an amount of from 0% to 97% by weight of the first solid composition,
a second solid composition comprising:
   (c) an oxidizable substrate in an amount of from 3% to 100% by weight of the second composition selected from:
      (i) negatively charged halogens and their derivatives, or
      (ii) pseudohalogens and their derivatives; and
   (d) optionally at least one inert filler in an amount of from 0% to 97% by weight of the second composition, and
a third solid composition comprising:
   (e) at least one oxidising agent in an amount of from 3% to 100% by weight of the third composition; and
   (f) optionally at least one inert filler in an amount of from 0% to 97% by weight of the third composition.

If the second solid composition comprises a buffer system then it may be present in an amount of up to 40% by wt of the second composition and the amount of inert filler is reduced accordingly to up to 57% by wt of the second composition.

The above weight % of components specified above is not intended to be limiting on the invention and is included to provide an example. The amount of each components in each composition may be varied depending on the specific components selected and to fit the end use of the desired product.

The kit provides a multiple container product which allows the multiple active ingredients to be stored for maximum shelf life. Preferably each of the first, second and third (if present) composition is contained in a sachet, container or capsule. Preferably, wherein if the container is a bottle it is made from either plastic or glass. The capsule may be made of a biodegradable material. The solid composition when in a single formulation may also be contained in a sachet, container or capsule.

It is possible for the solid composition to exist in a single container. In particular, if one or more of the ingredients used is immobilised or encapsulated it is possible for the ingredients to form part of the same solid composition. The use of separate sachets, containers or capsules allows the reactive ingredients to be kept apart and thus increases the shelf life of the solid composition. It is also ensures that there is no unwanted reactions between reagents.

The sachet, container or capsule may hold the first, second and third solid compositions. When the kit comprises separate sachets, preferably each of the first, second and third solid compositions is present in an amount of from 0.2 g to 2.0 g (as is desirable for pharmaceutical products). Preferably wherein the total weight of each of the single, two or three sachets/capsules/containers in the kit is 0.6 to 6.0g. However, the amount of composition per sachet, container or capsule is not limited to this weight and can be varied as appropriate. Optionally, if a 2 g sachet is prepared, and if the buffer system is present, the buffer system is present in an amount from 0.5 to 0.9.g.

Preferably wherein the shelf life of the first, second and third solid compositions exceeds two years (as is desirable for pharmaceutical and cosmetic products and in most other industries).

To prepare an aqueous system (also known herein as an aqueous solution), the user simply adds the solid compositions to water in the form of a single formulation, or multiple compositions (as described above). If necessary, the aqueous solution comprising the solid compositions is stirred or shaken to dissolve the ingredients and to activate the reaction.

The water may be tap water, distilled water, deionised water, reverse osmosis water or bottled water. It may be water which is held in a large container such as a tanker. The volume of water to be treated is not restricted. It is even possible to create a continuous flow by controlled addition of the solid composition as described herein into flowing water.

The amount of ingredients held in the sachet, container or capsule will depend on the requirement of the aqueous system and the volume of water. However, for ease of handling and accurate dosing (such as into sachets and capsules) smaller amount of the products are typically used.

As described above, the first, second and third solid compositions may be held in separate sachets or containers. When preparing the aqueous solution, the first solid composition is added to the water, followed by the second solid composition and then optionally the third composition, if present. The aqueous solution may then be mixed. The solution may be mixed for up to 30 minutes, for example 25 minutes, 20 minutes or 15 minutes. After mixing, the solution may then left for a period of from 1 minute to up to 20 minutes prior to use. The inventors have found that by allowing the aqueous solution to settle after mixing, the stability of the hypothiocyanite ions and the shelf-life of the solution is increased.

The antibacterial solution, disinfectant, antifungal solution and antiviral solution prepared according to the present invention are used in methods for:
(i) cleaning, washing and disinfecting materials including surfaces;
(ii) the preparation of solutions intended for pharmaceutical and cosmetic products;
(iii) introducing into the air and applying on to surfaces to kill pathogens.
(iv) creating fine spray or mist prior to inhalation by human and/or animals.

Another purpose of the invention also concerns methods of using the antibacterial solution, disinfectant, antifungal solution or antiviral solution described herein, alone or in combination with other anti-infectious, antimicrobial, antiviral, antibiotic, antifungal agents, or preservatives for disinfection and sanitization of materials, surfaces, equipment and medical devices. For example, the invention can be used in operating theatres to disinfect the surfaces or equipment therein.

Alternatively, the aqueous solutions can be used for air treatment, by decontamination of the air (passive) such as in planes, ambient decontamination (active) and environmental clean-up.

Also described herein is the use of the solid composition of the invention described herein or part of the aqueous system, alone or in combination with other anti-infectious, antimicrobial, antiviral, antibiotic, antifungal agents, or preservatives for the treatment of foods or drinking water, recreational water and water used for subsequent antimicrobial applications.

The invention also concerns a method of using the antibacterial solution, disinfectant, antifungal solution or antiviral solution as described herein, alone or in combination with other anti-infectious, antimicrobial, antiviral for the removal of pathogens in agriculture in the form of a spray or mist on fields of flowers or crops or used in greenhouses or used in storage facilities for perishable goods.

The composition may be used alone or in combination with other anti-infectious, antimicrobial, antiviral, antibiotic, antifungal agents, or preservatives for its co-use with cleaning or disinfection agents.

The improved stability and shelf-life of the prepared aqueous solution also allows for the product to be used where it is desirable to use large quantities of solution or where there is a requirement for the aqueous solution containing the active species to be stable for longer periods of time. For example, the aqueous solution can be prepared in large quantities and used to water plants in fields (where it would typically take a farmer several hours to do so), to wash vegetables using a constant flow of the aqueous solution or to spray as a mist in the air in a food warehouse.

The present invention provides a method of sterilising water comprising the steps of:
using the kit defined herein, wherein the first, second and optionally third solid compositions are added to the water to be sterilised.

The present invention provides a method of preparing an antibacterial solution, disinfectant, antifungal solution and antiviral solution comprising the steps of:
using the kit as defined herein, wherein the first, second and optionally third solid compositions are added to the water; and
optionally adding further other anti-infectious, antimicrobial, antiviral, antibiotic, antifungal agents, preservatives or disinfection agents.

The target concentration of the active species for solutions prepared in accordance with the present invention is 50-95ppm. This is known to be the most stable initial production level and also a strongly reactive level in terms of anti-pathogen activity, especially in solution. Below 25ppm there is very little anti-pathogen activity, and killed pathogens can soon be replaced by growth of existing pathogens.

In air the target activity will be 25-50ppm when it is seen to be effective at pathogen reduction and at a safe level for human and animal inhalation or skin contact.

The present inventors have also found that increased levels of active species is also possible of up to 120 ppm with the inclusion of a buffer (as demonstrated in the Examples and Figures below).

### EXAMPLES

### Kit preparation

The following kit was prepared:
- 1st container (0.5 g total weight): 0.2 g LPO, 0.118 g NaSCN, 0.182 g microcrystalline cellulose
- 2nd container (0.5 g total weight): 0.104 g CaO₂, 0.396 g microcrystalline cellulose

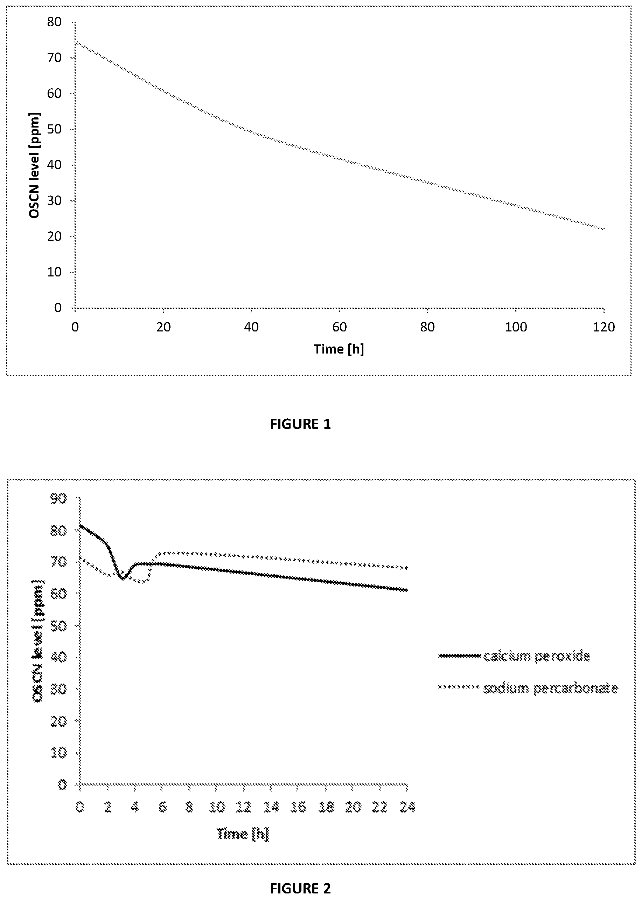

The test was performed with the above powder amount per 500 mL of water. Figure 1 demonstrates the scale up of this experiment in which the amount of hypothiocyanite ions during 120 hours of storage at 4 °C in a 5 L scale was measured. The graph shows that the product retains satisfactory hypothiocyanite levels in excess of 40 hours.

Figure 2 shows a comparison of calcium peroxide and sodium percarbonate effects on of hypothiocyanite stability. Figure 2 shows that use of sodium percarbonate provides lower levels in the first 5 hours period which then rises and stays higher in comparison to a reaction utilizing calcium peroxide for the whole 24 hours storage time. However, both achieve satisfactory levels of stability.

The following kits were also prepared:

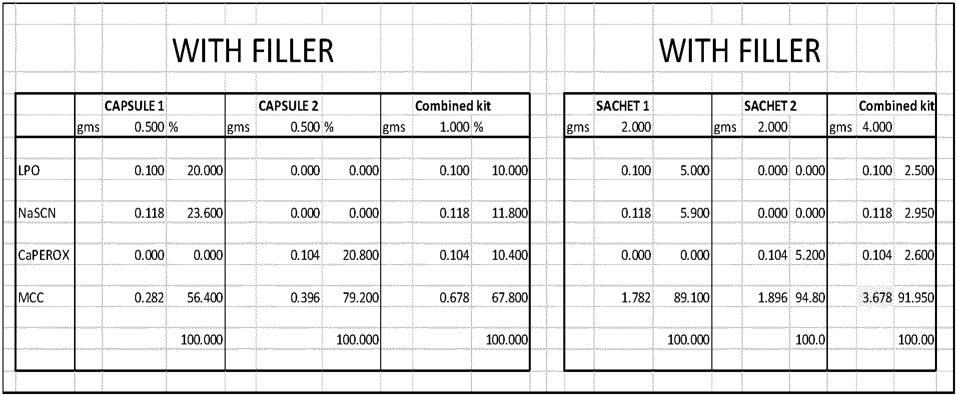

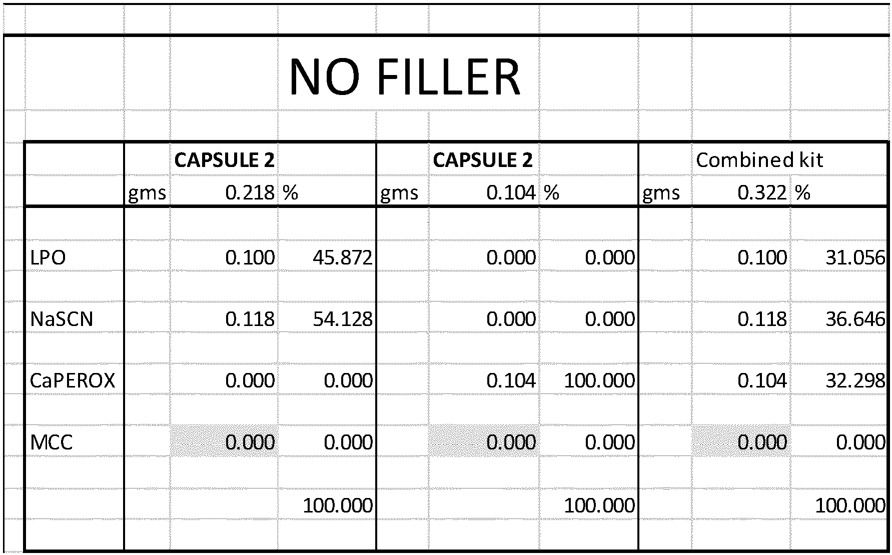

### Buffer optimisation & stability

Buffer systems were trialled in order to establish whether higher hypothiocyanite levels could be obtained. The kit prepared above (discussed with reference to Figures 1 and 2) was tested, and 6.5 mMol of a citric acid : trisodium citrate buffer was included.

The ingredients of container 1 above was mixed for 10 minutes . The ingredients of container 2 (containing the solid composition described above 0.104 g CaO₂, 0.396 g microcrystalline cellulose, plus buffer ) was added, and volume made up to the total 330ml with water. Container 2 was mixed for 15 minutes. Notably a significant pH drop following container 2 addition in the 6.5mM buffered test was observed.

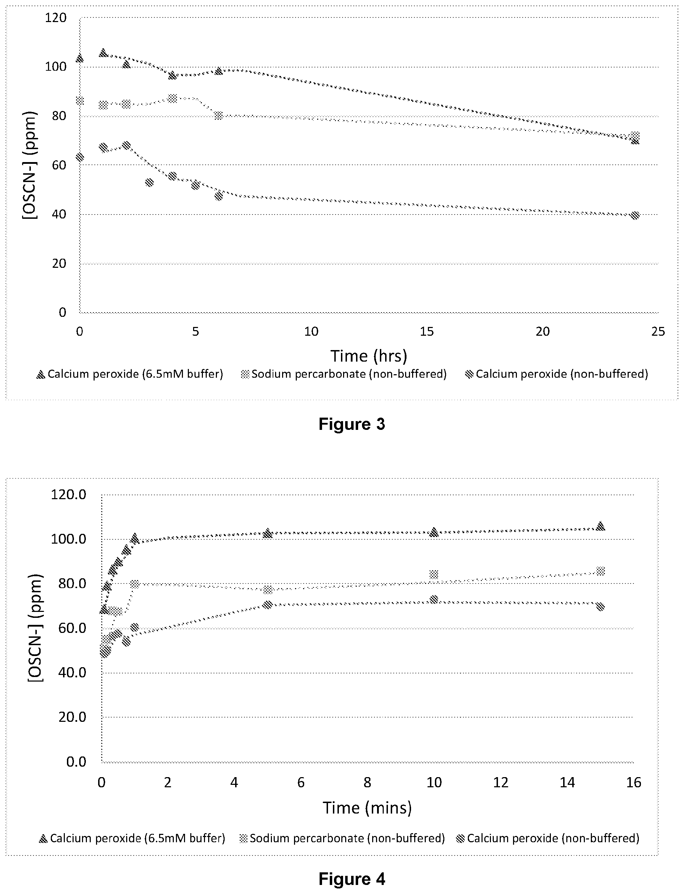

Figure 3 shows increased hypothiocyanite levels were produced when using a buffered calcium peroxide.

Figure 4 demonstrates that after resting of the aqueous solution comprising the buffer system post calcium peroxide/ sodium percarbonate addition and post mixing, the hypothiocyanite ppm increased and then stabilised.

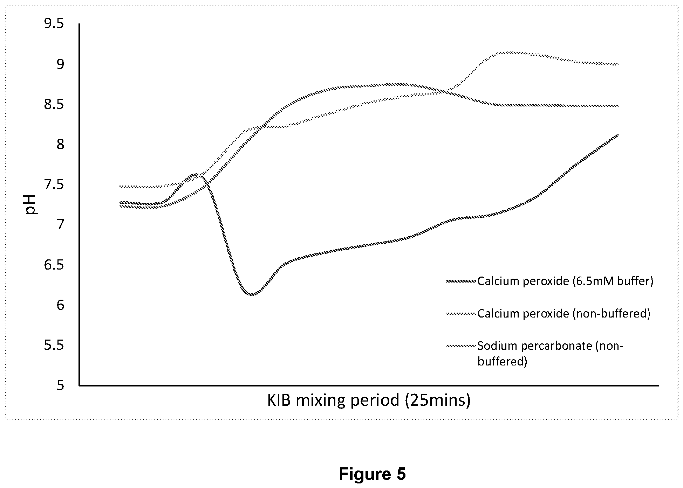

Figure 5 shows the pH changes throughout the mixing period (25 minutes total). Note that KIB is the name of the product/aqueous solution prepared in accordance with the present invention.

The above results show that a buffer may be useful for the present invention because it allows for an optimum pH profile of the reaction. The benefits gained include longer stability (up to 24hours), of the hypothiocyanite in solution. This longer period of stability will be useful in areas such as agriculture to spray a field/crop to destroy bacterial or viral problems or to clean or sterilise hospital theatres or waiting areas where sterility is desirable.

## Claims

1. A kit comprising:
a first solid composition comprising:
(a) a peroxidase enzymatic catalysing agent, wherein the peroxidase enzymatic catalysing agent is selected from Lactoperoxidase, Myeloperoxidase, Eosinophil peroxidase, Urea peroxidase and plant-derived peroxidases;
(b) an oxidizable substrate selected from:
(i) chlorides such as sodium chloride, iodides such as potassium iodide and sodium iodide; or
(ii) sodium thiocyanate, potassium thiocyanate, sodium bisulfite, sodium hydrosulfite, sodium metabisulfite, sodium nitrite, potassium nitrite, sodium hypochlorite and combinations thereof;
(c) optionally at least one inert filler; and
(d) a buffer system, wherein the buffer system is selected from citric acid: trisodium citrate; calcium lactate : citric acid; sodium L (+)-tartrate dehydrate: citric acid; calcium lactate : DL-malate : malic acid and Sodium L (+)- tartrate : tartaric acid
a second solid composition comprising:
(e) at least one oxidising agent, wherein the oxidising agent is selected from calcium peroxide, sodium peroxide, sodium percarbonate and the combination of dextrose and glucose oxidase; and
(f) optionally at least one inert filler.

2. A kit comprising:
a first solid composition comprising:
(a) a peroxidase enzymatic catalysing agent, wherein the peroxidase enzymatic catalysing agent is selected from Lactoperoxidase, Myeloperoxidase, Eosinophil peroxidase, Urea peroxidase and plant-derived peroxidases;
(b) optionally at least one inert filler,
a second solid composition comprising:
(c) an oxidizable substrate selected from:
(i) chlorides such as sodium chloride, iodides such as potassium iodide and sodium iodide; or
(ii) sodium thiocyanate, potassium thiocyanate, sodium bisulfite, sodium hydrosulfite, sodium metabisulfite, sodium nitrite, potassium nitrite, sodium hypochlorite;
(d) optionally at least one inert filler;
(e) a buffer system, wherein the buffer system is selected from citric acid: trisodium citrate; calcium lactate : citric acid; sodium L (+)-tartrate dehydrate: citric acid; calcium lactate : DL-malate : malic acid and Sodium L (+)- tartrate : tartaric acid and
a third solid composition comprising:
(f) at least one oxidising agent, wherein the oxidising agent is selected from calcium peroxide, sodium peroxide, sodium percarbonate and the combination of dextrose and glucose oxidase; and
(g) optionally at least one inert filler.

3. The kit according to claim 1 or claim 2, wherein the solid composition comprises at least one inert filler which is selected from microcrystalline cellulose, calcium carbonate, dextrose monohydrate, magnesium stearate, dicalcium phosphate, lactose powder, multifunctional starch, partially depolymerized cellulose, partially pre-gelatinized starches, highly functional starch, bentonite, silica and combinations thereof.

4. The kit according to any preceding claim, wherein the oxidizable substrate and/or the oxidizing agent is encapsulated.

5. The kit according to any preceding claim, wherein the kit does not comprise:
(i) a coagulant;
(ii) a thickening agent, preferably wherein the formulation does not contain a clay such as bentonite; and/or
(iii) a flocculant.

6. The kit according to any preceding claim, wherein each of the first and second and optionally third composition is contained separately in a sachet, container or capsule.

7. The kit according to any preceding claim, wherein, wherein the
(a) peroxidase enzymatic catalysing agent is Lactoperoxidase,
(b) oxidizable substrate is sodium thiocyanate and/or potassium thiocyanate and/or potassium iodide;
(c) at least one oxidising agent is calcium peroxide;
(d) at least one inert filler is microcrystalline cellulose; and
(e) wherein the kit comprises a buffer system.

8. A method of sterilising water comprising the step of:
using the kit according to any one of claims 1 to 7, wherein the first, second and optionally third solid compositions are added to the water to be sterilised.

9. A method of preparing an antibacterial solution, disinfectant, antifungal solution and antiviral solution comprising the steps of:
using the kit according to any one of claims 1 to 7, wherein the first, second and optionally third solid compositions are added to water; and
optionally adding further other anti-infectious, antimicrobial, antiviral, antibiotic, antifungal agents, preservatives or disinfection agents.

10. A method of using the antibacterial solution, disinfectant antifungal solution or antiviral solution according to claim 9 for:
(i) cleaning, washing and disinfecting materials including surfaces, equipment and medical devices;
(ii) the preparation of solutions intended for pharmaceutical and cosmetic products;
(iii) introducing into the air and applying on to surfaces to kill pathogens; and
(iv) creating fine spray or mist prior to inhalation by human and/or animals.

11. A method of using the antibacterial solution, disinfectant antifungal solution or antiviral solution according to claim 9, alone or in combination with other anti-infectious, antimicrobial, antiviral for the removal of pathogens in agriculture in the form of a spray or mist on fields of flowers or crops or used in greenhouses or used in storage facilities for perishable goods.

## Patentansprüche

1. Kit, umfassend:
eine erste feste Zusammensetzung, umfassend:
(a) einen Peroxidaseenzym-Katalysator, wobei der Peroxidaseenzym-Katalysator aus Lactoperoxidase, Myeloperoxidase, eosinophiler Peroxidase, Harnstoffperoxidase und pflanzenbasierten Peroxidasen ausgewählt ist;
(b) ein oxidierbares Substrat, ausgewählt aus:
(i) Chloriden wie Natriumchlorid, Iodiden wie Kaliumiodid und Natriumiodid; oder
(ii) Natriumthiocyanat, Kaliumthiocyanat, Natriumbisulfit, Natriumhydrosulfit, Natriummetabisulfit, Natriumnitrit, Kaliumnitrit, Natriumhypochlorit und Kombinationen davon;
(c) optional mindestens einen inerten Füllstoff; und
(d) ein Puffersystem, wobei das Puffersystem aus Citronensäure:Trinatriumcitrat; Calciumlactat:Citronensäure; Natrium-L-(+)-tartrat-Dehydrat:Citronensäure; Calciumlactat:DL-Malat:Äpfelsäure und Natrium-L-(+)-tartrat:Weinsäure ausgewählt ist;
eine zweite feste Zusammensetzung, umfassend:
(e) mindestens ein Oxidationsmittel, wobei das Oxidationsmittel aus Calciumperoxid, Natriumperoxid, Natriumpercarbonat und der Kombination von Dextrose und Glucoseoxidase ausgewählt ist; und
(f) optional mindestens einen inerten Füllstoff.

2. Kit, umfassend:
eine erste feste Zusammensetzung, umfassend:
(a) einen Peroxidaseenzym-Katalysator, wobei der Peroxidaseenzym-Katalysator aus Lactoperoxidase, Myeloperoxidase, eosinophiler Peroxidase, Harnstoffperoxidase und pflanzenbasierten Peroxidasen ausgewählt ist;
(b) optional mindestens einen inerten Füllstoff,
eine zweite feste Zusammensetzung, umfassend:
(c) ein oxidierbares Substrat, ausgewählt aus:
(i) Chloriden wie Natriumchlorid, Iodiden wie Kaliumiodid und Natriumiodid; oder
(ii) Natriumthiocyanat, Kaliumthiocyanat, Natriumbisulfit, Natriumhydrosulfit, Natriummetabisulfit, Natriumnitrit, Kaliumnitrit, Natriumhypochlorit;
(d) optional mindestens einen inerten Füllstoff;
(e) ein Puffersystem, wobei das Puffersystem aus Citronensäure:Trinatriumcitrat; Calciumlactat:Citronensäure; Natrium-L-(+)-tartrat-Dehydrat:Citronensäure; Calciumlactat:DL-Malat:Äpfelsäure und Natrium-L-(+)-tartrat:Weinsäure ausgewählt ist; und
eine dritte feste Zusammensetzung, umfassend:
(f) mindestens ein Oxidationsmittel, wobei das Oxidationsmittel aus Calciumperoxid, Natriumperoxid, Natriumpercarbonat und der Kombination von Dextrose und Glucoseoxidase ausgewählt ist; und
(g) optional mindestens einen inerten Füllstoff.

3. Kit nach Anspruch 1 oder Anspruch 2, wobei die feste Zusammensetzung mindestens einen inerten Füllstoff umfasst, der aus mikrokristalliner Cellulose, Calciumcarbonat, Dextrose-Monohydrat, Magnesiumstearat, Dicalciumphosphat, Lactosepulver, multifunktionaler Stärke, partiell depolymerisierter Cellulose, partiell vorgelierten Stärken, hochfunktionaler Stärke, Bentonit, Kieselsäure und Kombinationen davon ausgewählt ist.

4. Kit nach einem der vorhergehenden Ansprüche, wobei das oxidierbare Substrat und/oder das Oxidationsmittel verkapselt ist.

5. Kit nach einem der vorhergehenden Ansprüche, wobei das Kit nicht umfasst:
(i) ein Koagulationsmittel;
(ii) ein Verdickungsmittel, vorzugsweise wobei die Formulierung keinen Ton wie Bentonit enthält; und/oder
(iii) ein Flockungsmittel.

6. Kit nach einem der vorhergehenden Ansprüche, wobei jede der ersten und der zweiten und optional der dritten Zusammensetzung separat in einem Beutel, einem Behälter oder einer Kapsel enthalten ist.

7. Kit nach einem der vorhergehenden Ansprüche, wobei
(a) der Peroxidaseenzym-Katalysator Lactoperoxidase ist,
(b) das oxidierbare Substrat Natriumthiocyanat und/oder Kaliumthiocyanat und/oder Kaliumiodid ist;
(c) das mindestens eine Oxidationsmittel Calciumperoxid ist;
(d) der mindestens eine inerte Füllstoff mikrokristalline Cellulose ist; und
(e) wobei das Kit ein Puffersystem umfasst.

8. Verfahren zum Sterilisieren von Wasser, umfassend den Schritt:
Verwenden des Kits nach einem der Ansprüche 1 bis 7, wobei die erste, die zweite und optional die dritte feste Zusammensetzung dem zu sterilisierenden Wasser zugegeben werden.

9. Verfahren zur Herstellung einer antibakteriellen Lösung, eines Desinfektionsmittels, einer antimykotischen Lösung und einer antiviralen Lösung, umfassend die Schritte:
Verwenden des Kits nach einem der Ansprüche 1 bis 7, wobei die erste, die zweite und gegebenenfalls dritte feste Zusammensetzung Wasser zugegeben werden; und
optional Zugeben weiterer anderer antiinfektiöser, antimikrobieller, antiviraler, antibiotischer, antimykotischer Mittel, Konservierungsmittel oder Desinfektionsmittel.

10. Verfahren zur Verwendung der antibakteriellen Lösung, antimykotischen Desinfektionslösung oder antiviralen Lösung nach Anspruch 9 zum:
(i) Reinigen, Waschen und Desinfizieren von Materialien, einschließlich Oberflächen, Ausrüstungen und medizinischer Vorrichtungen;
(ii) zur Herstellung von Lösungen, die für pharmazeutische und kosmetische Erzeugnisse vorgesehen sind;
(iii) Einbringen in die Luft und Aufbringen auf Oberflächen, um Pathogene abzutöten; und
(iv) Erzeugen von feinem Spray oder Nebel vor der Inhalation durch Menschen und/oder Tiere.

11. Verfahren zur Verwendung der antibakteriellen Lösung, antimykotischen Desinfektionslösung oder antiviralen Lösung nach Anspruch 9 allein oder in Kombination mit anderen Antiinfektiva, Antimikrobiotika, Viruziden zur Entfernung von Pathogenen in der Landwirtschaft in Form eines Sprays oder Nebels auf Blumen- oder Getreiedefeldern oder zur Verwendung in Gewächshäusern oder zur Verwendung in Lagereinrichtungen für leicht verderbliche Waren.

## Revendications

1. Kit, comprenant :
une première composition solide comprenant :
(a) un agent catalyseur enzymatique de peroxydase, dans lequel l'agent catalyseur enzymatique de peroxydase est choisi parmi la lactoperoxydase, la myéloperoxydase, la peroxydase éosinophile, la peroxydase urée et les peroxydases d'origine végétale ;
(b) un substrat oxydable choisi parmi :
(i) des chlorures tels que le chlorure de sodium, des iodures tels que l'iodure de potassium et l'iodure de sodium ; ou
(ii) le thiocyanate de sodium, le thiocyanate de potassium, le bisulfite de sodium, l'hydrosulfite de sodium, le métabisulfite de sodium, le nitrite de sodium, le nitrite de potassium, l'hypochlorite de sodium et leurs combinaisons ;
(c) éventuellement au moins une charge inerte ; et
(d) un système de tampon, dans lequel le système de tampon est choisi parmi l'acide citrique : le citrate trisodique ; le lactate de calcium : l'acide citrique ; le L (+)-tartrate de sodium déshydraté : l'acide citrique ; le lactate de calcium : le DL-malate : l'acide malique et le L (+)-tartrate de sodium : l'acide tartrique
une deuxième composition solide comprenant :
(e) au moins un agent oxydant, dans lequel l'agent oxydant est choisi parmi le peroxyde de calcium, le peroxyde de sodium, le percarbonate de sodium et la combinaison de dextrose et de glucose oxydase ; et
(f) éventuellement au moins une charge inerte.

2. Kit, comprenant :
une première composition solide comprenant :
(a) un agent catalyseur enzymatique de peroxydase, dans lequel l'agent catalyseur enzymatique de peroxydase est choisi parmi la lactoperoxydase, la myéloperoxydase, la peroxydase éosinophile, la peroxydase urée et les peroxydases d'origine végétale ;
(b) éventuellement au moins une charge inerte,
une deuxième composition solide comprenant :
(c) un substrat oxydable choisi parmi :
(i) des chlorures tels que le chlorure de sodium, des iodures tels que l'iodure de potassium et l'iodure de sodium ; ou
(ii) le thiocyanate de sodium, le thiocyanate de potassium, le bisulfite de sodium, l'hydrosulfite de sodium, le métabisulfite de sodium, le nitrite de sodium, le nitrite de potassium, l'hypochlorite de sodium ;
(d) éventuellement au moins une charge inerte ;
(e) un système de tampon, dans lequel le système de tampon est choisi parmi l'acide citrique : le citrate trisodique ; le lactate de calcium : l'acide citrique ; le L (+)-tartrate de sodium déshydraté : l'acide citrique ; le lactate de calcium : le DL-malate : l'acide malique et le L (+)-tartrate de sodium : l'acide tartrique et
une troisième composition solide comprenant :
(f) au moins un agent oxydant, dans lequel l'agent oxydant est choisi parmi le peroxyde de calcium, le peroxyde de sodium, le percarbonate de sodium et la combinaison de dextrose et de glucose oxydase ; et
(g) éventuellement au moins une charge inerte.

3. Kit selon la revendication 1 ou la revendication 2, dans lequel la composition solide comprend au moins une charge inerte qui est choisie parmi la cellulose microcristalline, le carbonate de calcium, le dextrose monohydraté, le stéarate de magnésium, le phosphate dicalcique, la poudre de lactose, l'amidon multifonctionnel, la cellulose partiellement dépolymérisée, les amidons partiellement prégélatinisés, l'amidon hautement fonctionnel, la bentonite, la silice et leurs combinaisons.

4. Kit selon l'une quelconque des revendications précédentes, dans lequel le substrat oxydable et/ou l'agent oxydant est encapsulé.

5. Kit selon l'une quelconque des revendications précédentes, dans lequel le kit ne comprend pas :
(i) un coagulant ;
(ii) un agent épaississant, de préférence dans lequel la formulation ne contient pas d'argile telle que la bentonite ; et/ou
(iii) un floculant.

6. Kit selon une quelconque revendication précédente, dans lequel chacune des première et deuxième et éventuellement troisième compositions est contenue séparément dans un sachet, un récipient ou une capsule.

7. Kit selon une quelconque revendication précédente, dans lequel, dans lequel
(a) l'agent catalyseur enzymatique de peroxydase est la lactoperoxydase,
(b) le substrat oxydable est le thiocyanate de sodium et/ou le thiocyanate de potassium et/ou l'iodure de potassium ;
(c) au moins un agent oxydant est le peroxyde de calcium ;
(d) au moins une charge inerte est de la cellulose microcristalline ; et
(e) dans lequel le kit comprend un système de tampon.

8. Procédé de stérilisation de l'eau comprenant l'étape consistant à :
utiliser le kit selon l'une quelconque des revendications 1 à 7, dans lequel les première, deuxième et éventuellement troisième compositions solides sont ajoutées à l'eau devant être stérilisée.

9. Procédé de préparation d'une solution antibactérienne, d'un désinfectant, d'une solution antifongique et d'une solution antivirale comprenant les étapes consistant à :
utiliser le kit selon l'une quelconque des revendications 1 à 7, dans lequel les première, deuxième et éventuellement troisième compositions solides sont ajoutées à l'eau ; et
ajouter éventuellement d'autres agents anti-infectieux, antimicrobiens, antiviraux, antibiotiques, antifongiques, conservateurs ou désinfectants.

10. Procédé d'utilisation de la solution antibactérienne, de la solution désinfectante antifongique ou de la solution antivirale selon la revendication 9 pour :
(i) nettoyer, laver et désinfecter des matériaux, y compris les surfaces, l'équipement et les dispositifs médicaux ;
(ii) préparer des solutions destinées aux produits pharmaceutiques et cosmétiques ;
(iii) introduire dans l'air et appliquer sur les surfaces pour tuer les agents pathogènes ; et
(iv) créer une fine pulvérisation ou un brouillard avant l'inhalation par les humains et/ou les animaux.

11. Procédé d'utilisation de la solution antibactérienne, de la solution désinfectante antifongique ou de la solution antivirale selon la revendication 9, seule ou en combinaison avec d'autres anti-infectieux, antimicrobiens, antiviraux pour l'élimination d'agents pathogènes en agriculture sous forme de pulvérisation ou de brouillard sur les champs de fleurs ou de cultures ou utilisée dans les serres ou utilisée dans les installations de stockage de denrées périssables.
